(19) Europäisches Patentamt
European Patent Office
Office européen des brevets

(11) **EP 3 753 585 A1**

(12) **EUROPEAN PATENT APPLICATION**
published in accordance with Art. 153(4) EPC

(43) Date of publication:
**23.12.2020 Bulletin 2020/52**

(51) Int Cl.:
*A61L 9/01* (2006.01)  *A01N 59/16* (2006.01)
*A01N 59/20* (2006.01)  *B60R 13/02* (2006.01)
*C08J 7/04* (2020.01)  *C09D 5/14* (2006.01)
*C09D 7/61* (2018.01)  *C09D 183/00* (2006.01)
*C09D 201/00* (2006.01)  *E04F 13/08* (2006.01)

(21) Application number: 19784922.7

(22) Date of filing: 26.03.2019

(86) International application number:
**PCT/JP2019/012716**

(87) International publication number:
**WO 2019/198482 (17.10.2019 Gazette 2019/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.04.2018 JP 2018076978**

(71) Applicant: **Shin-Etsu Chemical Co., Ltd.
Tokyo 100-0004 (JP)**

(72) Inventors:
• **FURUDATE, Manabu
Kamisu-shi, Ibaraki 314-0102 (JP)**
• **INOUE, Tomohiro
Kamisu-shi, Ibaraki 314-0102 (JP)**

(74) Representative: **Angerhausen, Christoph
Boehmert & Boehmert
Anwaltspartnerschaft mbB
Pettenkoferstrasse 22
80336 München (DE)**

(54) **INTERIOR MATERIAL HAVING DEODORANT, ANTIMICROBIAL SURFACE LAYER AND PRODUCTION METHOD THEREOF**

(57) Provided are an interior material having a surface layer as a thin film with a high transparency and exhibiting deodorant and antimicrobial properties; and a production method thereof. The interior material has a surface layer containing (i) titanium oxide particles and (ii) antimicrobial metal-containing alloy particles. The interior material is such that an antimicrobial metal(s) contained in the (ii) antimicrobial metal-containing alloy particles is at least one kind of metal selected from the group consisting of silver, copper and zinc. The production method of the interior material of the present invention includes a step of applying a dispersion liquid containing the (i) titanium oxide particles and the (ii) antimicrobial metal-containing alloy particles to a surface of the interior material.

**Description**

TECHNICAL FIELD

The present invention relates to an interior material having a deodorant, antimicrobial surface layer. Particularly, the invention relates to an interior material having a surface layer that has a high transparency and a deodorant antimicrobial property; and a production method thereof.

BACKGROUND ART

In recent years, "safety and security" as well as "health and comfort" in the living space are demanded by the consumers, and materials having deodorant and antimicrobial effects are now desired for the purpose of controlling harmful volatile organic compounds (VOC) released from livingware-related products and buildings, and controlling unpleasant odors closely associated with daily lives such as the sweaty smell, the aging body odor, the smell of cigarette and the odor of food waste; and for the purpose of preventing contamination by microorganisms such as bacteria and fungi (molds).

Examples of a deodorant method using a deodorant agent include a chemical deodorant method, a physical deodorant method, a sensory deodorant method and a biological deodorant method; these methods are used differently based on intended purposes. The chemical deodorant method is to eliminate odors by causing chemical reactions between odor-causing substances and deodorant components, and enables odor elimination with a high selectivity with respect to a particular odor-causing substance(s). The physical deodorant method is to remove odor-causing substances from the air via physical adsorption, and relatively easily enables the simultaneous adsorption of multiple odor-causing substances with one adsorbent. As such adsorbent, there are used, for example, an activated carbon, zeolite, silica gel, alumina, titania and cyclodextrin. The sensory deodorant method is a method where odors are sensuously made insensible by performing, for example, masking or pairing with the aid of an aromatic component(s). This deodorant method differs from other deodorant methods in that it does not remove odor-causing substances from the air i.e. it can be said that this deodorant method cannot bring about any health-related effects. The biological deodorant method is a method where the generation of odor itself is restricted by controlling the proliferation of microorganisms as the source of odor generation.

As for a spray-type deodorant agent, there are known deodorant methods that are each performed alone or deodorant methods that are performed in combination. However, the deodorant effects of these methods and the persistence thereof have never been satisfactory.

In view of the characteristics of each deodorant method, the physical deodorant method is preferred in terms of eliminating various odors present in the living space; it is more preferred that the physical deodorant method be combined with other deodorant methods depending on location and situation.

For example, the sweaty smell is generated as a result of bacteria being proliferated by sweat, and then with such bacteria decomposing, for example, sebum mixed with sweat. The toilet smell contains ammonia as its main component, the ammonia being generated as a result of bacteria being proliferated by urine adhering to the toilet and its surrounding areas, and with such bacteria then decomposing the urine. Therefore, since controlling the proliferation of the bacteria is effective in controlling the generation of odors, a combination of the physical deodorant method and the biological deodorant method is more effective as they are capable of eliminating the odors and restricting the odor generation itself.

Agents produced by adding antimicrobial agents to adsorbents conventionally used in the physical deodorant method haven been commercialized as antimicrobial deodorant agents. However, in many cases, the deodorant antimicrobial effects of these antimicrobial deodorant agents are insufficient, and most products do not exhibit an antifungal effect. Further, these adsorbents are often in the form of particles or a powder, and cannot be diffused or sprayed in the air accordingly. Thus, it is difficult to achieve an immediate effectivity as it takes time for the odors to come into contact with and then be adsorbed to the adsorbent. In addition, it was also difficult to impart a deodorant antimicrobial effect by adhering adsorbents to, for example, interior or exterior architectural materials for buildings, furniture, fibrous products such as clothes and curtains as well as electric appliances without impairing the design features thereof.

Antimicrobial-antifungal agents can be roughly categorized into organic agents and inorganic agents. Organic synthetic antimicrobial-antifungal agents that have been frequently used in the past are inexpensive, and are effective even when used in a small amount. However, in many cases, these organic synthetic antimicrobial-antifungal agents are only effective on certain types of microorganisms (narrow antimicrobial spectrum), and the effects thereof may vary significantly in cases of gram-negative bacteria, gram-positive bacteria, fungi and the like. Further, the problems with these organic synthetic antimicrobial-antifungal agents are such that resistant bacteria can easily occur, a poor heat resistance is exhibited, and a low persistence is exhibited though a superior immediate effectivity is observed. Moreover, since concerns have been increasingly raised on the impact on the human body and environment, inorganic antimicrobial agents are gradually gaining dominance.

As an inorganic antimicrobial-antifungal agent, there is mainly employed a material with metal ions such as silver ions, copper ions or zinc ions being supported on a support; examples of such support include zeolite, silica gel, calcium phosphate and zirconium phosphate. As compared to an organic agent, an inorganic antimicrobial-antifungal agent has, for example, a feature of being effective on a wider range of microorganisms (wider antimicrobial spectrum), and a feature of exhibiting a high thermal stability. However, since an inorganic antifungal agent has a weak antifungal effect, organic antifungal agents are mainly used even nowadays as antifungal agents.

Here, the following patent documents 1 to 6 are listed as relevant prior art documents.

PRIOR ART DOCUMENTS

Patent documents

Patent document 1: Japanese Unexamined Patent Application Publication (Translation of PCT Application) No. 2003-533588
Patent document 2: JP-A-2003-113392
Patent document 3: JP-A-2001-070423
Patent document 4: JP-A-2001-037861
Patent document 5: JP-A-2001-178806
Patent document 6: JP-A-2005-318999

SUMMARY OF THE INVENTION

Problems to be solved by the invention

Thus, it is an object of the present invention to provide an interior material having a surface layer as a thin film with a high transparency and exhibiting deodorant and antimicrobial properties; and a method for producing such interior material.

Means to solve the problems

The inventors of the present invention diligently conducted a series of studies to achieve the abovementioned objectives, and completed the invention as follows. That is, the inventors found that a surface layer containing deodorant titanium oxide particles and antimicrobial metal-containing alloy particles was able to exhibit high deodorant and antimicrobial properties.

The interior material of the present invention has a surface layer containing deodorant titanium oxide particles and antimicrobial metal-containing alloy particles, thereby exhibiting higher deodorant and antimicrobial properties than ever.

In this way, the present invention is to provide the following interior material having a surface layer with a deodorant antimicrobial property; and a method for producing the same.

Here, in this specification, the term "antimicrobial property" may refer to a property for restricting the proliferation of microorganisms including bacteria and fungi (molds).

[1] An interior material having a surface layer containing (i) titanium oxide particles and (ii) antimicrobial metal-containing alloy particles.
[2] The interior material according to [1], wherein an antimicrobial metal(s) contained in the (ii) antimicrobial metal-containing alloy particles is at least one kind of metal selected from the group consisting of silver, copper and zinc.
[3] The interior material according to [2], wherein the (ii) antimicrobial metal-containing alloy particles at least contain silver.
[4] The interior material according to any one of [1] to [3], wherein the antimicrobial metal(s) contained in the (ii) antimicrobial metal-containing alloy particles is in an amount of 1 to 100% by mass with respect to a total mass of the alloy particles.
[5] The interior material according to any one of [1] to [4], wherein a dispersed particle size of a particle mixture of the (i) titanium oxide particles and the (ii) antimicrobial metal-containing alloy particles is 5 to 100 nm in terms of a 50% cumulative distribution diameter $D_{50}$ on volume basis that is measured by a dynamic light scattering method using a laser light.

[6] The interior material according to any one of [1] to [5], wherein the surface layer further contains a binder.

[7] The interior material according to [6], wherein the binder is a silicon compound-based binder.

[8] The interior material according to any one of [1] to [7], wherein the interior material is a material selected from the group consisting of an interior architectural material, a vehicular interior material, a material for household furniture and a material for electric appliances.

[9] A method for producing the interior material according to [1], comprising a step of applying a dispersion liquid containing the (i) titanium oxide particles and the (ii) antimicrobial metal-containing alloy particles to a surface of the interior material.

[10] The method for producing the interior material according to [9], wherein the dispersion liquid containing the (i) titanium oxide particles and the (ii) antimicrobial metal-containing alloy particles is applied via spray coating, flow coating, dip coating, spin coating, Meyer bar coating, gravure coating, knife coating, kiss coating, die coating and/or film transfer.

Effects of the invention

According to the present invention, there can be easily formed a thin film (surface layer) having a high transparency and exhibiting deodorant and antimicrobial properties; and there can be achieved, for example, an effect of controlling harmful volatile organic compounds (VOC) released from livingware-related products and buildings as well as unpleasant odors closely associated with daily lives such as the sweaty smell, the aging body odor, the smell of cigarette and the odor of food waste, and an effect of preventing contamination by microorganisms such as bacteria and fungi (molds), without impairing the design features of a product.

MODE FOR CARRYING OUT THE INVENTION

The preset invention is described in greater detail hereunder.

<Deodorant antimicrobial agent>

A deodorant antimicrobial agent contained in a surface layer of the interior material of the present invention is comprised of a particle mixture of at least two kinds of particles of (i) titanium oxide particles and (ii) antimicrobial metal-containing alloy particles. When applying them, it is preferred that at least two kinds of particles of (i) the titanium oxide particles and (ii) the antimicrobial metal-containing alloy particles be at first dispersed in an aqueous dispersion medium. As described later, this can be produced by mixing at least two kinds of particle dispersion liquids of a titanium oxide particle dispersion liquid and an antimicrobial metal-containing alloy particle dispersion liquid that have been separately prepared.

Titanium oxide particle dispersion liquid

As crystalline phases of titanium oxide particles, there are generally known three of them which are the rutile-type, anatase-type and brookite-type. It is preferred that there be used those mainly composed of the anatase-type or rutile-type. Here, the expression "mainly composed" refers to an occupancy of usually not smaller than 50% by mass, preferably not smaller than 70% by mass, even more preferably not smaller than 90% by mass, or even 100% by mass in all the crystals of the titanium oxide particles.

As titanium oxide particles, there may be employed those with metal compounds of platinum, gold, palladium, iron, copper, nickel or the like being supported on titanium oxide particles, those doped with elements such as tin, nitrogen, sulfur, carbon and transition metals, or even a titanium oxide useful as a photocatalyst, for the purpose of improving an deodorant property of the particles.

It is more preferable to use a titanium oxide intended as a photocatalyst, because an even higher deodorant and antimicrobial effect can be achieved when irradiated with lights.

A titanium oxide useful as a photocatalyst may be a general photocatalytic titanium oxide, or a visible light responsive photocatalytic titanium oxide configured to be able to respond to a visible light of 400 to 800 nm.

As the aqueous dispersion medium for the titanium oxide particle dispersion liquid, an aqueous solvent is normally used, and it is preferred that water be used. Further, there may also be used a water-soluble organic solvent mixable with water, and a mixed solvent prepared by mixing water and a water-soluble organic solvent at any ratio. As water, preferred are, for example, a deionized water, a distilled water and a pure water. Moreover, as the water-soluble organic solvent, preferred are, for example, alcohols such as methanol, ethanol and isopropanol; glycols such as ethylene glycol; and glycol ethers such as ethylene glycol monomethyl ether, ethylene glycol monoethyl ether and propylene glycol-n-propyl ether. As the aqueous dispersion medium, any one kind of them may be used alone, or two or more kinds of them may be used in combination. If using the mixed solvent, it is preferred that a ratio of the water-soluble organic solvent in the mixed solvent be larger than 0% by mass, but not larger than 50% by mass; more preferably larger than 0% by mass, but not larger than 20% by mass; even more preferably larger than 0% by mass, but not larger than 10% by mass.

As for a dispersed particle size of the titanium oxide particles in the titanium oxide particle dispersion liquid, a 50% cumulative distribution diameter $D_{50}$ on volume basis that is measured by a dynamic light scattering method using a laser light (possibly referred to as "average particle size" hereunder) is preferably 5 to 30 nm, more preferably 5 to 20 nm. This is because when the average particle size is smaller than 5 nm, an insufficient deodorant capability may be exhibited; and when the average particle size is greater than 30 nm, the dispersion liquid may turn non-transparent. Here, as a device for measuring the average particle size, there may be used, for example, ELSZ-2000ZS (by Otsuka Electronics Co., Ltd.), NANOTRAC UPA-EX150 (by Nikkiso Co., Ltd.), and LA-910 (by HORIBA, Ltd.).

It is preferred that the concentration of the titanium oxide particles in the titanium oxide particle dispersion liquid be 0.01 to 30% by mass, particularly preferably 0.5 to 20% by mass, in terms of ease in producing a later-described titanium oxide-alloy thin film having a given thickness.

Here, a method for measuring the concentration of the titanium oxide particle dispersion liquid may be such that part of the titanium oxide particle dispersion liquid is taken as a sample, followed by heating it at 105°C for three hours so as to volatilize the solvent, and then calculating the concentration in accordance with the following formula based on the mass of the non-volatile content (titanium oxide particles), and the mass of the sampled titanium oxide particle dispersion liquid before heating.

$$\text{Concentration of titanium oxide particle dispersion liquid (\%)} = [\text{mass of non-volatile content (g)/mass of titanium oxide particle dispersion liquid before heating (g)}] \times 100$$

Antimicrobial metal-containing alloy particle dispersion liquid

In the present invention, the alloy particles are comprised of at least two kinds of metal components, and contain at least one kind of antimicrobial metal.

The term "antimicrobial metal" refers to metals that are harmful to microorganisms such as bacteria and fungi (molds), but are relatively less harmful to the human body; examples of such metals include silver, copper, zinc, platinum, palladium, nickel, aluminum, titanium, cobalt, zirconium, molybdenum and tungsten that are known to reduce the viable count of Staphylococcus aureus and E. coli in a specification test for antimicrobial products JIS Z 2801 when used as metal component particles to coat a film (references 1 and 2 as below).

Reference 1: Miyano, Iron and steel, 93(2007)1, 57-65
Reference 2: H. Kawakami, ISIJ Intern., 48(2008)9, 1299-1304

It is preferred that the alloy particles used in the interior material of the present invention contain at least one of these metals, particularly preferably at least one of silver, copper and zinc. More specifically, the alloy particles used in the interior material of the present invention may be those comprised of combinations of metal components, such as silver-copper, silver-palladium, silver-platinum, silver-tin, gold-copper, silver-nickel, silver-antimony, silver-copper-tin, gold-copper-tin, silver-nickel-tin, silver-antimony-tin, platinum-manganese, silver-titanium, copper-tin, cobalt-copper, zinc-magnesium, silver-zinc, copper-zinc and silver-copper-zinc.

There are no particular restrictions on the components in the alloy particles other than the antimicrobial metal(s); examples of such components may include gold, antimony, tin, sodium, magnesium, silicon, phosphorus, sulfur, potassium, calcium, scandium, vanadium, chromium, manganese, iron, gallium, germanium, arsenic, selenium, yttrium, niobium, technetium, ruthenium, rhodium, indium, tellurium, cesium, barium, hafnium, tantalum, rhenium, osmium, iridium, mercury, thallium, lead, bismuth, polonium, radium, lanthanum, cerium, praseodymium, neodymium, promethium, samarium, europium, gadolinium, actinium and thorium. Any one of them may be used alone, or two or more of them may be used in combination.

The antimicrobial metal(s) in the alloy particles are contained in an amount of 1 to 100% by mass, preferably 10 to 100% by mass, more preferably 50 to 100% by mass, with respect to the total mass of the alloy particles. This is because when the antimicrobial metal(s) are in an amount of smaller than 1% by mass with respect to the total mass of the alloy particles, an insufficient antimicrobial capability may be exhibited.

As the aqueous dispersion medium for the alloy particle dispersion liquid, an aqueous solvent is normally used; preferred are water, a water-soluble organic solvent mixable with water, and a mixed solvent prepared by mixing water and a water-soluble organic solvent at any ratio. As water, preferred are, for example, a deionized water, a distilled water and a pure water. Further, examples of the water-soluble organic solvent include alcohols such as methanol, ethanol, n-propanol, 2-propanol, n-butanol, 2-butanol, tert-butanol, ethylene glycol, diethylene glycol and polyethylene glycol; glycol ethers such as ethylene glycol monomethyl ether, ethylene glycol monoethyl ether, ethylene glycol monobutyl ether, diethylene glycol monomethyl ether, diethylene glycol monoethyl ether, diethylene glycol monobutyl ether and propylene glycol-n-propyl ether; ketones such as acetone and methyl ethyl ketone; water-soluble nitrogen-containing compounds such as 2-pyrrolidone and N-methylpyrrolidone; and ethyl acetate. Any one of them may be used alone, or two or more of them may be used in combination.

As for a dispersed particle size of the alloy particles in the alloy particle dispersion liquid, a 50% cumulative distribution diameter $D_{50}$ on volume basis that is measured by a dynamic light scattering method using a laser light (possibly referred to as "average particle size" hereunder) is preferably not larger than 200 nm, more preferably not larger than 100 nm, even more preferably not larger than 70 nm. There are no particular restrictions on the lower limit of the average particle size, and theoretically, even those having the minimum particle size enabling antimicrobial property may be used. However, practically, it is preferred that the average particle size be not smaller than 1 nm. Further, it is not preferable when the average particle size is greater than 200 nm, because the dispersion liquid may turn non-transparent. Here, as a device for measuring the average particle size, there may be used, for example, ELSZ-2000ZS (by Otsuka Electronics Co., Ltd.), NANOTRAC UPA-EX150 (by Nikkiso Co., Ltd.), and LA-910 (by HORIBA, Ltd.).

There are no particular restrictions on the concentration of the alloy particles in the alloy particle dispersion liquid. However, in general, the lower the concentration is, the better the dispersion stability becomes. Thus, it is preferred that the concentration be 0.0001 to 10% by mass, more preferably 0.001 to 5% by mass, even more preferably 0.01 to 1% by mass. It is not preferable when the concentration is lower than 0.0001% by mass, because the productivity of the interior material will decrease in a significant manner.

Titanium oxide-alloy particle mixed dispersion liquid

As described above, a titanium oxide-alloy particle mixed dispersion liquid for use in the production of the interior material of the present invention is obtained by mixing the titanium oxide particle dispersion liquid and the antimicrobial metal-containing alloy particle dispersion liquid that have been produced separately.

Here, as for a dispersed particle size of the mixture of the titanium oxide particles and the antimicrobial metal-containing alloy particles in the titanium oxide-alloy particle mixed dispersion liquid, a 50% cumulative distribution diameter $D_{50}$ on volume basis that is measured by a dynamic light scattering method using a laser light (possibly referred to as "average particle size" hereunder) is 5 to 100 nm, preferably 5 to 30 nm, more preferably 5 to 20 nm. This is because when the average particle size is smaller than 5 nm, an insufficient deodorant capability may be exhibited; and when the average particle size is greater than 100 nm, the dispersion liquid may turn non-transparent.

Here, a device for measuring the average particle size of the particle mixture of the titanium oxide particles and the alloy particles is described as above.

In addition, the titanium oxide-alloy particle mixed dispersion liquid used in the interior material of the present invention may also contain a later-described binder.

A binder may be added to the titanium oxide-alloy particle mixed dispersion liquid for the purpose of making it easier for the dispersion liquid to be applied to the surfaces of various members that are described later, and the purpose of making it easier for the particles to adhere to these surfaces. Examples of such binder include metal compound-based binders containing silicon, aluminum, titanium, zirconium or the like; and organic resin-based binders containing a fluororesin, an acrylic resin, a urethane resin or the like.

A mass ratio between the binder and the titanium oxide-alloy particles [binder/(titanium oxide particles+alloy particles)] is 0.01 to 99, preferably 0.05 to 20, more preferably 0.1 to 9, even more preferably 0.4 to 2.5; it is preferred that the binder be added at a mass ratio within these ranges. This is because when this mass ratio is lower than 0.01, the titanium oxide particles may adhere to the surfaces of various members in an insufficient manner; and when such mass ratio is greater than 99, an insufficient deodorant capability and antimicrobial capability may be exhibited.

Particularly, in order to obtain a titanium oxide-alloy thin film having a high deodorant capability, antimicrobial capability and transparency, it is especially preferred that a silicon compound-based binder be added to the titanium oxide-alloy particle mixed dispersion liquid at a compounding ratio (mass ratio of silicon compound:(titanium oxide particles+alloy particles)) of 1:99 to 99:1, more preferably 10:90 to 90:10, even more preferably 30:70 to 70:30. Here, a "silicon compound-based binder" refers to a colloid dispersion, solution or emulsion of a silicon compound that is provided in a way such that a solid or liquid silicon compound is contained in an aqueous dispersion medium; specific examples of such silicon compound-based binder include a colloidal silica (preferable particle size 1 to 150 nm); solutions of silicates, such as a solution of silicate; silane, siloxane hydrolysate emulsions; silicone resin emulsions; and emulsions of copolymers of silicone resins and other resins, such as a silicone-acrylic resin copolymer and a silicone-urethane resin copolymer.

Further, if a binder for improving film forming capability is to be added, it is preferred that an aqueous binder solution to be added be prepared at first, followed by adding this aqueous binder solution to the titanium oxide-alloy particle mixed dispersion liquid whose concentration has already been adjusted to a desired concentration as above.

Furthermore, a water-soluble organic solvent and a surfactant, for example, may be added to the titanium oxide-alloy particle mixed dispersion liquid and the coating liquid prepared by adding a binder to such dispersion liquid, for the purpose of improving a coating property to the interior material.

As the water-soluble organic solvent, preferred are, for example, alcohols such as methanol, ethanol and isopropanol; glycols such as ethylene glycol; and glycol ethers such as ethylene glycol monomethyl ether, ethylene glycol monoethyl ether and propylene glycol-n-propyl ether. If using the water-soluble organic solvent, it is preferred that a ratio of the water-soluble organic solvent in the dispersion liquid or the coating liquid be larger than 0, but not larger than 50% by mass; more preferably larger than 0, but not larger than 20% by mass; even more preferably larger than 0, but not larger than 10% by mass.

Examples of the surfactant include anionic surfactants such as fatty acid sodium salt, alkylbenzene sulfonate, higher alcohol sulfate ester salt and polyoxyethylene alkyl ether sulfate; cationic surfactants such as alkyltrimethyl ammonium salt, dialkyldimethyl ammonium salt, alkyldimethylbenzyl ammonium salt and quaternary ammonium salt; amphoteric surfactants such as alkylamino fatty acid salt, alkyl betaine and alkylamine oxide; nonionic surfactants such as polyoxyethylene alkyl ether, polyoxyethylene alkylphenol ether, alkyl glucoside, polyoxyethylene fatty acid ester, sucrose fatty acid ester, sorbitan fatty acid ester, polyoxyethylene sorbitan fatty acid ester and fatty acid alkanolamide; and polymeric surfactants. Among these examples, nonionic surfactants are preferred in terms of stability of the dispersion liquid.

If using the surfactant, it is preferred that the concentration of the surfactant be larger than 0, preferably in a range of 0.001 to 5.0 parts by mass, more preferably 0.01 to 1.0 parts by mass, even more preferably 0.05 to 0.5 parts by mass, per a total of 100 parts by mass of all the components in the titanium oxide-alloy particle mixed dispersion liquid and the coating liquid (i.e. a total of 100 parts by mass of the above titanium oxide particles, alloy particles, non-volatile impurities, binder, solvent and surfactant).

<Method for producing deodorant antimicrobial agent>

A method for producing the deodorant antimicrobial agent used in the interior material of the present invention includes the following steps (1) to (6); the deodorant antimicrobial agent is eventually obtained in the form (of a mixed liquid) where (i) the titanium oxide particles and (ii) the antimicrobial metal-containing alloy particles are dispersed in the aqueous dispersion medium.

(1) Step of producing a peroxotitanic acid solution from a raw material titanium compound, a basic substance, hydrogen peroxide and an aqueous dispersion medium.
(2) Step of obtaining the titanium oxide particle dispersion liquid by heating the peroxotitanic acid solution produced in the step (1) at 80 to 250°C under a controlled pressure.
(3) Step of producing a solution containing a raw material antimicrobial metal compound, and a solution containing a reductant for reducing such metal compound.
(4) Step of producing an alloy particle dispersion liquid by mixing the solutions produced in the step (3) which are the solution containing the raw material antimicrobial metal compound, and the solution containing the reductant for reducing such metal compound.
(5) Step of washing the alloy particle dispersion liquid produced in the step (4) with an aqueous dispersion medium by a membrane filtration method.
(6) Step of mixing the titanium oxide particle dispersion liquid obtained in the step (2) and the alloy particle dispersion liquid obtained in the step (5).

The steps (1) and (2) are steps for producing the titanium oxide particle dispersion liquid.
The steps (3) to (5) are steps for producing the alloy particle dispersion liquid. While there are various physical and chemical methods, these production steps particularly employ a liquid phase reduction method which is a chemical method having advantages in terms of ease in adjusting synthesis conditions, wider controllable ranges of, for example, composition, particle size and particle size distribution, and productivity of the alloy particles. In such liquid phase reduction method, alloy particles are to be precipitated by mixing a reductant into a solution containing at least two kinds of metal ions serving as alloy raw materials. At that time, by allowing a protective agent of the alloy particles to coexist in the reaction system, the dispersibility of the alloy particles in the solvent can also be further improved.

The step (6) is a step for finally producing the titanium oxide-alloy particle mixed dispersion liquid having a deodorant and antimicrobial property, by mixing the titanium oxide particle dispersion liquid obtained in the step (2) and the alloy particle dispersion liquid obtained in the step (5).

Each step is described in detail hereunder.

Step (1):

In the step (1), the peroxotitanic acid solution is produced by reacting the raw material titanium compound, the basic substance and hydrogen peroxide in the aqueous dispersion medium.

As a method for producing the peroxotitanic acid solution, there may be employed a method where the basic substance is added to the raw material titanium compound in the aqueous dispersion medium to obtain titanium hydroxide, followed by eliminating impurity ions other than the metal ions contained, and then adding hydrogen peroxide thereto so as to obtain peroxotitanic acid; or a method where after adding hydrogen peroxide to the raw material titanium compound, the basic substance is then added thereto to obtain a peroxotitanium hydrate, followed by eliminating impurities other than the metal ions contained, and then further adding hydrogen peroxide thereto so as to obtain peroxotitanic acid.

Here, examples of the raw material titanium compound include titanium chlorides; inorganic acid salts such as nitrates and sulfates; organic acid salts such as formic acid, citric acid, oxalic acid, lactic acid and glycolic acid; and titanium hydroxides precipitated as a result of performing hydrolysis by adding alkalis to the aqueous solutions of these compounds. Any one of them may be used alone, or two or more of them may be used in combination. Particularly, as the raw material titanium compound, it is preferred that a titanium chloride(s) ($TiCl_3$, $TiCl_4$) be used.

As the aqueous dispersion medium, an aqueous dispersion medium similar to that in the titanium oxide particle dispersion liquid is used such that the aforementioned composition will be achieved. Here, the concentration of the raw material titanium compound aqueous solution comprised of the raw material titanium compound and the aqueous dispersion medium is not higher than 60% by mass, particularly preferably not higher than 30% by mass. While the lower limit of such concentration may be appropriately selected, it is preferred that the concentration be not lower than 1% by mass in general.

The basic substance is used to smoothly turn the raw material titanium compound into titanium hydroxide; examples of such basic substance include hydroxides of alkali metals or alkali earth metals, such as sodium hydroxide and potassium hydroxide; and amine compounds such as ammonia, alkanolamine and alkylamine. The basic substance is added in an amount at which the raw material titanium compound aqueous solution will have a pH level of not lower than 7, particularly 7 to 10. Here, the basic substance may also be used in the form of an aqueous solution having an appropriate concentration when combined with the aqueous dispersion medium.

Hydrogen peroxide is used to convert the raw material titanium compound or titanium hydroxide into peroxotitanium i.e. a titanium oxide compound containing a Ti-O-O-Ti bond, and is normally used in the form of a hydrogen peroxide water. It is preferred that hydrogen peroxide be added in an amount of 1.5 to 20 times larger than the substance quantity of titanium in terms of mole. Further, in the reaction where hydrogen peroxide is added to turn the raw material titanium compound or titanium hydroxide into peroxotitanic acid, it is preferred that a reaction temperature be 5 to 80°C, and that a reaction time be 30 min to 24 hours.

The peroxotitanic acid solution thus obtained may also contain an alkaline substance or an acidic substance for the purpose of pH adjustment or other purposes. Here, examples of the alkaline substance include ammonia, sodium hydroxide, calcium hydroxide and alkylamine. Examples of the acidic substance include inorganic acids such as sulfuric acid, nitric acid, hydrochloric acid, carbonic acid, phosphoric acid and hydrogen peroxide; and organic acids such as formic acid, citric acid, oxalic acid, lactic acid and glycolic acid. In this case, it is preferred that the peroxotitanic acid solution obtained have a pH level of 1 to 9, particularly preferably 4 to 7 in terms of safety in handling.

Step (2):

In the step (2), the peroxotitanic acid solution obtained in the step (1) is subjected to a hydrothermal reaction at a temperature of 80 to 250°C, preferably 100 to 250°C for 0.01 to 24 hours under a controlled pressure. An appropriate reaction temperature is 80 to 250°C in terms of reaction efficiency and reaction controllability; as a result, the peroxotitanic acid will be converted into titanium oxide particles. Here, the expression "under a controlled pressure" refers to a state where when the reaction temperature employed is greater than the boiling point of the dispersion medium, pressure will be applied in an appropriate manner such that the reaction temperature can be maintained; as well as a state where when the reaction temperature employed is not higher than the boiling point of the dispersion medium, atmospheric pressure will be used for control. Here, the pressure is normally about 0.12 to 4.5 MPa, preferably about 0.15 to 4.5 MPa, more preferably 0.20 to 4.5 MPa. The reaction time is preferably 1 min to 24 hours. The titanium oxide particle dispersion liquid is obtained via this step (2).

While it is preferred that the particle size of the titanium oxide particles thus obtained be within the aforementioned range(s), the particle size can be controlled by adjusting the reaction conditions. For example, the particle size can be reduced by shortening the reaction time and a temperature rising time.

• Step (3):

In the step (3), produced are the solution with the raw material antimicrobial metal compound being dissolved in an aqueous dispersion medium; and the solution with the reductant for reducing such raw material antimicrobial metal compound being dissolved in an aqueous dispersion medium.

As a method for producing these solutions, there may be employed a method where the raw material antimicrobial metal compound and the reductant for reducing such raw material antimicrobial metal compound are individually and separately added to an aqueous dispersion medium, followed by performing stirring so as to allow them to be dissolved therein. There are no particular restrictions on a stirring method as long as the method employed enables a uniform dissolution in the aqueous dispersion medium; a commonly available stirrer can be used.

Various antimicrobial metal compounds may be used as the raw material antimicrobial metal compound, examples of which include antimicrobial metal chlorides; inorganic acid salts such as nitrates and sulfates; organic acid salts such as formic acid, citric acid, oxalic acid, lactic acid and glycolic acid; and complex salts such as amine complex, cyano complex, halogeno complex and hydroxy complex. Any one of them may be used alone, or two or more of them may be used in combination. Particularly, it is preferred that chlorides and inorganic acid salts such as nitrates and sulfates be used.

There are no particular restrictions on the reductant; there can be used any kind of reductant capable of reducing the metal ions composing the raw material antimicrobial metal compound. Examples of the reductant include hydrazines such as hydrazine, hydrazine monohydrate, phenylhydrazine and hydrazinium sulfate; amines such as dimethylaminoethanol, triethylamine, octylamine and dimethylaminoborane; organic acids such as citric acid, ascorbic acid, tartaric acid, malic acid, malonic acid and formic acid; alcohols such as methanol, ethanol, isopropyl alcohol, ethylene glycol, diethylene glycol, triethylene glycol, tetraethylene glycol and benzotriazole; hydrides such as sodium borohydride, lithium borohydride, lithium triethylborohydride, lithium aluminum hydride, diisobutylaluminum hydride, tributyltin hydride, lithium tri(sec-butyl)borohydride, potassium tri(sec-butyl)borohydride, zinc borohydride and acetoxy sodium borohydride; pyrrolidones such as polyvinylpyrrolidone, 1-vinylpyrrolidone, N-vinylpyrrolidone and methylpyrrolidone; reducing sugars such as glucose, galactose, mannose, fructose, sucrose, maltose, raffinose and stachyose; and sugar alcohols such as sorbitol.

A protective agent may also be added to the solution with the reductant being dissolved in the aqueous dispersion medium. There are no particular restrictions on the protective agent as long as the protective agent employed is capable of preventing the alloy particles precipitated by reduction from agglutinating; there may be used a surfactant or an organic compound having a capability as a dispersant. Specific examples of the protective agent include surfactants such as anionic surfactants, cationic surfactants and nonionic surfactants; water-soluble polymer compounds such as polyvinylpyrrolidone, polyvinyl alcohol, polyethyleneimine, polyethylene oxide, polyacrylic acid and methylcellulose; aliphatic amine compounds such as ethanolamine, diethanolamine, triethanolamine and propanolamine; primary amine compounds such as butylamine, dibutylamine, hexylamine, cyclohexylamine, heptylamine, 3-butoxypropylamine, octylamine, nonylamine, decylamine, dodecylamine, hexadecylamine, oleylamine and octadecylamine; diamine compounds such as N,N-dimethylethylenediamine and N,N-diethylethylenediamine; and carboxylic acid compounds such as oleic acid.

As the aqueous dispersion medium (aqueous solvent), it is preferred that there be used water, a water-soluble organic solvent mixable with water, or a mixed solvent prepared by mixing water and a water-soluble organic solvent at any ratio. As water, preferred are, for example, a deionized water, a distilled water and a pure water. Further, examples of the water-soluble organic solvent include alcohols such as methanol, ethanol, isopropanol, n-propanol, 2-propanol, n-butanol, 2-butanol, tert-butanol, ethylene glycol and diethylene glycol; glycol ethers such as ethylene glycol monomethyl ether, ethylene glycol monoethyl ether, ethylene glycol monobutyl ether, diethylene glycol monomethyl ether, diethylene glycol monoethyl ether, diethylene glycol monobutyl ether and propylene glycol-n-propyl ether; ketones such as acetone and methyl ethyl ketone; water-soluble nitrogen-containing compounds such as 2-pyrrolidone and N-methylpyrrolidone; and ethyl acetate. As the aqueous dispersion medium, any one of them may be used alone, or two or more of them may be used in combination.

A basic substance or an acidic substance may be added to the aqueous dispersion medium. Examples of such basic substance include alkali metal hydroxides such as sodium hydroxide and potassium hydroxide; alkali metal carbonates such as sodium carbonate and potassium carbonate; alkali metal hydrogen carbonates such as sodium hydrogen carbonate and potassium hydrogen carbonate; alkali metal alkoxides such as potassium tert-butoxide, sodium methoxide and sodium ethoxide; alkali metal salts of aliphatic hydrocarbons such as butyl lithium; and amines such as triethylamine, diethylaminoethanol and diethylamine. Examples of the acidic substance include inorganic acids such as aqua regia, hydrochloric acid, nitric acid and sulfuric acid; and organic acids such as formic acid, acetic acid, chloroacetic acid, dichloroacetic acid, oxalic acid, trifluoroacetic acid and trichloroacetic acid.

There are no particular restrictions on the concentrations of the solution with the raw material antimicrobial metal compound being dissolved in the aqueous dispersion medium and the solution with the reductant for reducing such raw material antimicrobial metal compound being dissolved in the aqueous dispersion medium. However, there is a tendency that the lower these concentrations are, the smaller a primary particle size of each alloy particle formed will become. That is, it is preferred that a preferable concentration range(s) be determined based on the range of a target primary particle size.

There are no particular restrictions on the pH levels of the solution with the raw material antimicrobial metal compound being dissolved in the aqueous dispersion medium and the solution with the reductant for reducing such raw material antimicrobial metal compound being dissolved in the aqueous dispersion medium. It is preferred that the pH levels of these solutions be adjusted to preferable levels based on, for example, target molar ratios of the metals in the alloy particles and a target primary particle size.

Step (4):

In the step (4), the solution with the raw material antimicrobial metal compound being dissolved in the aqueous dispersion medium and the solution with the reductant for reducing such raw material antimicrobial metal compound being dissolved in the aqueous dispersion medium, which have been prepared in the step (3), are mixed to produce the alloy particle dispersion liquid.

There are no particular restrictions on a method for mixing these two solutions, as long as the method employed allows the two solutions to be uniformly mixed together. For example, there may be employed a method where the metal compound solution and the reductant solution are put into a reaction container before being stirred and mixed together; a method where stirring and mixing is performed in a way such that the reductant solution is delivered by drops into the metal compound solution already placed in a reaction container while stirring such metal compound solution; a method where stirring and mixing is performed in a way such that the metal compound solution is delivered by drops into the reductant solution already placed in a reaction container while stirring such reductant solution; or a method where the metal compound solution and the reductant solution are continuously supplied in constant amounts such that a reaction container or a microreactor, for example, may then be used to perform mixing.

There are no particular restrictions on a temperature at the time of preforming mixing; it is preferred that the temperature be adjusted to a preferable temperature based on, for example, target molar ratios of the metals in the alloy particles and a target primary particle size.

Step (5):

In the step (5), the alloy particle dispersion liquid produced in the step (4) is washed with an aqueous dispersion medium by a membrane filtration method.

As the aqueous dispersion medium, it is preferred that there be used water, a water-soluble organic solvent mixable with water, or a mixed solvent prepared by mixing water and a water-soluble organic solvent at any ratio. As water, preferred are, for example, a deionized water, a distilled water and a pure water. Further, examples of the water-soluble organic solvent include alcohols such as methanol, ethanol, isopropanol, n-propanol, 2-propanol, n-butanol, 2-butanol, tert-butanol, ethylene glycol and diethylene glycol; glycol ethers such as ethylene glycol monomethyl ether, ethylene glycol monoethyl ether, ethylene glycol monobutyl ether, diethylene glycol monomethyl ether, diethylene glycol monoethyl ether, diethylene glycol monobutyl ether and propylene glycol-n-propyl ether; ketones such as acetone and methyl ethyl ketone; water-soluble nitrogen-containing compounds such as 2-pyrrolidone and N-methylpyrrolidone; and ethyl acetate. As the water-soluble organic solvent, any one of them may be used alone, or two or more of them may be used in combination.

In the step (5), a membrane filtration method is used to wash and separate non-volatile impurities other than the alloy particles, such as components other than the metals in the raw material metal compound, the reductant and the protective agent, away from the alloy particle dispersion liquid produced in the step (4). It is preferred that washing be performed repeatedly until a mass ratio between the alloy particles and the non-volatile impurities in the alloy particle dispersion liquid (alloy particles/non-volatile impurities) has reached 0.01 to 10, more preferably 0.05 to 5, even more preferably 0.1 to 1. It is not preferable when the mass ratio is lower than 0.01, because there will be a large amount of impurities with respect to the alloy particles so that an antimicrobial, antifungal and deodorant properties imparted may not be fully exhibited; it is also not preferable when the mass ratio is greater than 10, because the dispersion stability of the alloy particles may deteriorate.

• Determination of metal component concentration in alloy particle dispersion liquid (ICP-OES)

The metal component concentration in the alloy particle dispersion liquid can be measured by appropriately diluting the alloy particle dispersion liquid with a pure water, and then introducing the diluted liquid into an inductively coupled plasma optical emission spectrometer (product name "Agilent 5110 ICP-OES" by Agilent Technologies, Inc.)

•Determination of non-volatile impurities other than metal components in alloy particle dispersion liquid

Here, the concentration of the non-volatile impurities other than the metal components in the alloy particle dispersion liquid can be calculated by subtracting the metal component concentration determined by the above ICP-OES from a non-volatile content concentration that is calculated based on a mass of non-volatile contents (alloy particles+non-volatile impurities) observed after the solvent has been volatilized as a result of heating part of the alloy particle dispersion liquid as a sample at 105°C for three hours, and a mass of the sampled alloy particle dispersion liquid before heating.

$$\text{Non-volatile impurity concentration (\%)} = [\text{mass of non-volatile content (g)/mass of}$$

$$\text{alloy particle dispersion liquid before heating (g)}] \times 100\text{-metal component concentration in alloy}$$

$$\text{particle dispersion liquid (\%)}$$

There are no particular restrictions on a membrane used in the membrane filtration method, as long as the membrane used is capable of separating the alloy particles and the non-volatile impurities other than the alloy particles from the alloy particle dispersion liquid. Examples of such membrane include a microfiltration membrane, an ultrafiltration membrane and a nanofiltration membrane. Among these membranes, filtration can be carried out using a membrane having a suitable pore size.
As a filtration method, there may also be employed any of, for example, centrifugal filtration, pressure filtration and cross-flow filtration.
As for the shape of the filtration membrane, there may be appropriately employed those of, for example, a hollow-fiber type, a spiral type, a tubular type or a flat membrane type.
There are no particular restrictions on the material of the filtration membrane, as long as the material employed has a durability against the alloy particle dispersion liquid. The material may be appropriately selected from, for example, organic films such as those made of polyethylene, tetrafluoroethylene, difluoroethylene, polypropylene, cellulose acetate, polyacrylonitrile, polyimide, polysulfone and polyether sulfone; and inorganic films such as those made of silica, alumina, zirconia and titania.
Specific examples of the abovementioned filtration membrane include microza (by Asahi Kasei Chemicals Corporation), Amicon Ultra (by Merck Millipore Corporation), Ultra filter (by Advantec Toyo Kaisha, Ltd.) and MEMBRALOX (by Nihon Pall Ltd.).

Step (6):

In the step (6), the titanium oxide particle dispersion liquid obtained in the step (2) and the alloy particle dispersion liquid obtained in the step (5) are mixed to produce the titanium oxide-alloy particle mixed dispersion liquid having a deodorant and antimicrobial property.
There are no particular restrictions on a mixing method, as long as the method employed allows the dispersion liquids to be uniformly mixed together; for example, mixing may be carried out by performing stirring using a commonly available stirrer.
A mixing ratio between the titanium oxide particle dispersion liquid and the alloy particle dispersion liquid is 1 to 100,000, preferably 10 to 10,000, even more preferably 20 to 1,000, in terms of a particle mass ratio between the titanium oxide particles and the alloy particles in each dispersion liquid (titanium oxide particles/alloy particles). It is not preferable when the mass ratio is lower than 1, because the deodorant capability will not be fully exhibited; it is also not preferable when the mass ratio is greater than 100,000, because the antimicrobial capability will not be fully exhibited.
As for a dispersed particle size of the mixture of the titanium oxide particles and the alloy particles in the titanium oxide-alloy particle mixed dispersion liquid, a 50% cumulative distribution diameter $D_{50}$ on volume basis that is measured by a dynamic light scattering method using a laser light (possibly referred to as "average particle size" hereunder) is defined as above.
Further, a device for measuring the average particle size is defined as above as well.
A total concentration of the titanium oxide particles, the alloy particles and the non-volatile impurities in the titanium oxide-alloy particle mixed dispersion liquid is preferably 0.01 to 20% by mass, particularly preferably 0.5 to 10% by mass, in terms of ease in producing a titanium oxide-alloy thin film having a given thickness, as described above. This total concentration can be adjusted in a manner such that when the total concentration is higher than a desired concentration, the total concentration can be lowered via dilution with the addition of an aqueous dispersion medium; when the total concentration is lower than a desired total concentration, the total concentration can be raised by volatilizing or filtrating away the aqueous dispersion medium.

Here, a method for measuring the concentration of the titanium oxide-alloy particle mixed dispersion liquid is such that part of the titanium oxide-alloy particle mixed dispersion liquid is taken as a sample, followed by heating it at 105°C for three hours so as to volatilize the solvent, and then calculating the concentration in accordance with the following formula based on the mass of the non-volatile contents (titanium oxide particles, alloy particles and non-volatile impurities), and the mass of the sampled titanium oxide-alloy particle mixed dispersion liquid before heating.

$$\text{Concentration of titanium oxide-alloy particle mixed dispersion liquid (\% by mass)} =$$

$$[\text{mass of non-volatile content (g)/mass of titanium oxide-alloy particle mixed dispersion liquid}$$

$$\text{before heating (g)}]\times100$$

<Interior material having surface layer containing titanium oxide-alloy particles>

The titanium oxide-alloy particle mixed dispersion liquid can be used to form a deodorant antimicrobial thin film (surface layer) on the surface of an interior material. The interior material may have various shapes depending on the purpose and the intended use thereof.

Here, in this specification, the term "interior material" refers to, for example, an interior architectural material such as a wall material, a wall paper, a ceiling material, a floor material, tiles, bricks, a wooden board, a resin board, a metallic plate, a tatami mat and a bathroom material for use in architectural structures; a vehicular interior material such as a wall material, a ceiling material, a floor material, a seat, a handrail and a hanging strap for use in an automobile and trains, for example; a material for household furniture and livingware-related products such as curtains, a blind, a rug, a partition board, a glass product, a mirror, a film, a desk, a chair, a bed and a storage rack; and a material for home electric appliances such as an air cleaner, an air conditioner, a refrigerator, a laundry machine, a personal computer, a printer, a tablet, a touch panel and a telephone set.

Here, as materials for various interior materials, there may be listed, for example, organic materials and inorganic materials.

Examples of organic materials include synthetic resin materials such as vinyl chloride resin (PVC), polyethylene (PE), polypropylene (PP), polycarbonate (PC), an acrylic resin, polyacetal, a fluororesin, a silicone resin, an ethylene-vinyl acetate copolymer (EVA), an acrylonitrile-butadiene rubber (NBR), polyethylene terephthalate (PET), polyethylene naphthalate (PEN), polyvinyl butyral (PVB), an ethylene-vinyl alcohol copolymer (EVOH), a polyimide resin, polyphenylene sulfide (PPS), polyetherimide (PEI), polyether ether imide (PEEI), polyether ether ketone (PEEK), a polyamide resin (PA), a melamine resin, a phenol resin and an acrylonitrile-butadiene-styrene (ABS) resin; natural materials such as natural rubbers; and semisynthetic materials of the above synthetic resin materials and natural materials. They may already be processed into desired shapes or structures such as those of a film, a sheet, a fibrous material, a fibrous product, other molded products or laminated products.

Non-metallic inorganic materials and metallic inorganic materials are, for example, included in the above inorganic materials.

Examples of non-metallic inorganic materials include glass, ceramics, stone materials and plasters. They may already be processed into various shapes such as those of tiles, glass products, mirrors, walls and design materials.

Examples of metallic inorganic materials include cast iron, steel, iron, iron alloys, stainless steel, aluminum, aluminum alloys, nickel, nickel alloys and zinc die-cast. They may already be plated with the above metallic inorganic materials or coated with the above organic materials, or even be used to plate the surfaces of the above organic materials or non-metallic inorganic materials.

As a method for forming the surface layer (deodorant antimicrobial thin film) on the surfaces of various interior materials, there may be employed, for example, a method where the titanium oxide-alloy particle mixed dispersion liquid or the coating liquid prepared by adding a binder to such titanium oxide-alloy particle mixed dispersion liquid is applied to the surface of any of the abovementioned interior materials via a method such as spray coating, flow coating, dip coating, spin coating, Meyer bar coating, reverse roll coating, gravure coating, knife coating, kiss coating or die coating, followed by performing drying or film transfer.

While a drying temperature after coating may be selected variously depending on the target base material to be coated, it is preferred that the drying temperature be 0 to 500°C, more preferably 5 to 200°C, even more preferably 10 to 150°C. This is because when the drying temperature is lower than 0°C, the dispersion liquid and/or the coating liquid may freeze and thus become unusable; and when the drying temperature is greater than 500°C, the deodorant antimicrobial property may deteriorate.

While a drying time after coating may be appropriately selected based on a coating method and the drying

**EP 3 753 585 A1**

temperature, it is preferred that the drying time be 10 sec to 72 hours, more preferably 20 sec to 48 hours. This is because it is not preferable when the drying time is shorter than 10 sec, as the deodorant antimicrobial thin film may adhere to the surface of the material in an insufficient manner; and it is also not preferable when the drying time is longer than three days, as there will be exhibited a poor economic efficiency in the production of the interior material.

While the thickness of the surface layer may be appropriately selected, it is preferred that the thickness be 10 nm to 10 $\mu$m, more preferably 20 nm to 5 $\mu$m, even more preferably 50 nm to 1 $\mu$m. This is because when the film thickness is smaller than 10 nm, there may be imparted an insufficient deodorant antimicrobial property; and when the film thickness is greater than 10 $\mu$m, the surface layer may be easily peeled off from the surface of the interior material.

The surface layer (deodorant antimicrobial thin film) formed in such manner is transparent, and is thus capable of imparting a favorable deodorant antimicrobial effect without impairing the design features of a product; an interior material having such surface layer formed thereon is capable of exhibiting effects such as a cleaning effect, a deodorizing effect and an antimicrobial effect of its own due to the deodorant antimicrobial action of the titanium oxide-alloy particles.

WORKING EXAMPLES

The present invention is described in detail hereunder with reference to working and comparative examples. However, the present invention is not limited to the following working examples.

Here, the "raw material antimicrobial metal compound" may be simply referred to as "raw material metal compound."

Various capability tests in the present invention were performed as follows.

(1) Deodorant capability test on interior material having titanium oxide-alloy thin film on its surface

In order to evaluate the deodorant capability of the interior material of the present invention that has the titanium oxide-alloy thin film on its surface, a coating liquid for evaluation that had been produced from the titanium oxide-alloy particle mixed dispersion liquid and a binder was taken by an amount of 1 g and then applied to an interior material cut into a 100 mm square, followed by drying the same so as to obtain a test piece. This test piece was then subjected to a test performed by a method according to a deodorant capability test described in JEC301 "The certification standards of SEK mark textile products" provided by (general incorporated association) Japan Textile Evaluation Technology Council, and was evaluated based on the following standards (Table 5). There were 10 kinds of odorous components targeted in this test which were ammonia, acetic acid, hydrogen sulfide, methyl mercaptan, trimethylamine, acetaldehyde, pyridine, isovaleric acid, nonenal and indole, as prescribed in the above standards.

- Very favorable (graded A) ⋯ seven or more kinds of gases showing an odorous component decline rate of not lower than 30%

- Favorable (graded B)⋯five or more kinds of gases showing an odorous component decline rate of not lower than 30%

- Slightly unfavorable (graded C)⋯three or more kinds of gases showing an odorous component decline rate of not lower than 30%

- Unfavorable (graded D)⋯two or fewer kinds of gases showing an odorous component decline rate of not lower than 30%

(2) Antimicrobial capability test on titanium oxide-alloy thin film

In order to evaluate the antimicrobial capability of the interior material of the present invention that has the titanium oxide-alloy thin film on its surface, the titanium oxide-alloy thin film was applied to the surface of a 50 mm square interior material in a manner such that the thin film would have a thickness of 100 nm thereon, thereby obtaining a test piece. This test piece was then subjected to a test performed by a method according to Japanese Industrial Standard JIS Z 2801:2012 "Antibacterial products-Test for antibacterial activity and efficacy," and was evaluated based on the following standards (Table 6).

- Very favorable (graded A) ⋯ all antimicrobial activity values were not lower than 4.0.
- Favorable (graded B) ⋯ all antimicrobial activity values were not lower than 2.0.
- Unfavorable (graded C) ⋯ antimicrobial activity values were lower than 2.0.

(3) Antifungal capability test on titanium oxide-alloy thin film

In order to evaluate the antifungal capability of the titanium oxide-alloy thin film, the titanium oxide-alloy thin film was applied to the surface of a 50 mm square interior material in a manner such that the thin film would have a thickness of 100 nm thereon, thereby obtaining a test piece. This test piece was then evaluated by a method according to Japanese Industrial Standard JIS Z 2911:2010 "Methods of test for fungus resistance," and even a test piece that had been subjected to cultivation for as long as eight weeks was evaluated.

- •    Very favorable (graded A) ··· fungus growth status 0 to 1
- •    Favorable (graded B) ··· fungus growth status 2 to 3
- •    Unfavorable (graded C) ··· fungus growth status 4 to 5

(4) Identification of crystalline phase of titanium oxide particles

The crystalline phase of the titanium oxide particles was identified by measuring the powder X-ray diffraction of a titanium oxide particle powder collected after drying a dispersion liquid of the titanium oxide particles obtained at 105°C for three hours, using a desktop X-ray diffraction device (product name "D2 PHASER" by Bruker AXS GmbH) (Table 1).

(5) Determination of alloy of alloy particles

The determination of whether the alloy particles were made of an alloy(s) was conducted by performing energy dispersive X-ray spectroscopic analysis under the observation of a scanning transmission electron microscope (STEM, ARM-200F by JEOL Ltd.). Specifically, the alloy particle dispersion liquid obtained was delivered by drops into a carbon grid for TEM observation, followed by performing drying so as to remove water, and then observing the particles under magnification. STEM-EDX mapping was then carried out by selecting several fields of view containing a plurality of particles having a shape regarded as an average shape. There, the particles were determined to be alloy particles and rated "○," when it was confirmed that all the metal components composing an alloy were detectable from one particle; the particles were determined to be non-alloy particles and rated "×," when the above status was not able to be confirmed.

(6) Average particle size $D_{50}$

An average particle size $D_{50}$ of the particles in the titanium oxide particle dispersion liquid, the alloy particle dispersion liquid as well as the mixture of the two kinds of particles which were the titanium oxide particles and the alloy particles, was calculated as a 50% cumulative distribution diameter on volume basis that is measured by a dynamic light scattering method using a laser light, with the aid of ELSZ-2000ZS (by Otsuka Electronics Co., Ltd.).

[Working example 1]

<Preparation of titanium oxide particle dispersion liquid>

After diluting a 36% by mass titanium chloride (IV) aqueous solution tenfold with a pure water, a 10% by mass ammonia water was then gradually added thereto to neutralize and hydrolyze the same, thereby obtaining a precipitate of titanium hydroxide. The precipitate-containing solution at that time had a pH level of 9. The precipitate obtained was then subjected to a deionization treatment where addition of pure water and decantation were performed repeatedly. A 3 5% by mass hydrogen peroxide water was then added to the deionized precipitate of titanium hydroxide in a manner such that a ratio of $H_2O_2$/Ti (molar ratio) would become 5, followed by stirring them at room temperature for 24 hours for sufficient reaction, thereby obtaining a yellow and transparent peroxotitanic acid solution (a).
The peroxotitanic acid solution (a) of an amount of 400 mL was put into a 500 mL autoclave to be hydrothermally processed for 90 min under a condition of 130°C, 0.5 MPa, followed by performing concentration control by adding a pure water thereto, thereby obtaining a titanium oxide particle dispersion liquid (A) (non-volatile content concentration 1.0% by mass) (Table 1).

<Preparation of silver-copper alloy particle dispersion liquid>

With ethylene glycol being used as a solvent, a raw material metal compound-containing solution (I) was produced by dissolving therein silver nitrate and copper nitrate trihydrate in a way such that a concentration as Ag would become 2.50 mmol/L, and a concentration as Cu would become 2.50 mmol/L (Table 2).

A reductant-containing solution (i) was obtained by mixing 55% by mass of ethylene glycol and 8% by mass of a pure water, as solvents; 2% by mass of potassium hydroxide as a basic substance; 20% by mass of hydrazine monohydrate and 5% by mass of dimethylaminoethanol, as reductants; and 10% by mass of polyvinylpyrrolidone as a reductant/protective agent.

A liquid obtained by rapidly mixing 2L of the raw material metal compound-containing solution (I) heated to 160°C in a reactor and 0.2 L of the reductant-containing solution (i) of a temperature of 25°C, was concentrated with the aid of an ultrafiltration membrane having a molecular weight cut-off of 10,000 (Microza by Asahi Kasei Chemicals Corporation) and washed with a pure water, thereby obtaining an alloy particle dispersion liquid ($\alpha$) (Table 3).

The titanium oxide particle dispersion liquid (A) and the alloy particle dispersion liquid ($\alpha$) were then mixed together in a way such that a mass ratio of the particles in each dispersion liquid (titanium oxide particles/alloy particles) would become 100, thereby obtaining a titanium oxide-alloy particle mixed dispersion liquid (e-1).

A silica-based binder (colloidal silica, product name: SNOWTEX20 by Nissan Chemical Corporation, average particle size 10 to 20 nm, aqueous solution with $SiO_2$ concentration of 20% by mass) was added to the titanium oxide-alloy particle mixed dispersion liquid (e-1) in a way such that $TiO_2/SiO_2$(mass ratio) would become 1.5, thereby obtaining a coating liquid for evaluation (E-1) (Table 4).

<Application to decorative gypsum board>

A decorative gypsum board used as a ceiling board was cut into pieces of sizes suitable for various tests, followed by using an air-spray gun (product model number "LPH-50-S9-10" by ANEST IWATA Corporation) to apply the coating liquid for evaluation (E-1) to the pieces with a discharge pressure of the air-spray gun being adjusted to 0.2 MPa. The pieces were then dried indoors at 20°C for 24 hours to obtain the interior material of the present invention. The surface of the interior material was then visually observed at a distance of 20 cm under visible light. As a result, no exterior abnormality was observed, and the interior material had a surface layer with a high transparency. The results of the deodorant capability test are summarized in Table 5; and the results of the antimicrobial capability test and the antifungal capability test are summarized in Table 6.

[Working example 2]

<Preparation of titanium oxide particle dispersion liquid>

A yellow and transparent peroxotitanic acid solution (b) was prepared in a similar manner as the working example 1, except that tin chloride (IV) was added to and dissolved into a 36% by mass titanium chloride (IV) aqueous solution in a way such that Ti/Sn (molar ratio) would become 20.

The peroxotitanic acid solution (b) of an amount of 400 mL was put into a 500 mL autoclave to be hydrothermally processed for 90 min under a condition of 150°C, 0.5 MPa, followed by performing concentration control by adding a pure water thereto, thereby obtaining a titanium oxide particle dispersion liquid (B) (non-volatile content concentration 1.0% by mass) (Table 1).

<Preparation of silver-palladium alloy particle mixed dispersion liquid>

An alloy particle dispersion liquid ($\beta$) (Table 3) was obtained in a similar manner as the working example 1, except that there was used a raw material metal compound-containing solution (II) (Table 2) with a pure water being a solvent, and with silver nitrate and a palladium nitrate dihydrate being dissolved therein in a way such that a concentration as Ag was 4.50 mmol/L, and a concentration as Pd was 0.50 mmol/L.

The titanium oxide particle dispersion liquid (B) and the alloy particle dispersion liquid ($\beta$) were then mixed together in a way such that a mass ratio of the particles in each dispersion liquid (titanium oxide particles/alloy particles) would become 200, thereby obtaining a titanium oxide-alloy particle mixed dispersion liquid (e-2).

A coating liquid for evaluation (E-2) was produced in a similar manner as the working example 1, except that there was used the titanium oxide-alloy particle mixed dispersion liquid (e-2) (Table 4).

<Application to melamine decorative board>

A melamine decorative board used as an interior partition board was cut into pieces of sizes suitable for various tests, followed by using the air-spray gun to apply the coating liquid for evaluation (E-2) to the pieces in a similar manner as the working example 1. The pieces were then dried in an oven at 50°C for three hours to obtain the interior material of the present invention. The surface of the interior material was then visually observed at a distance of 20 cm under visible light. As a result, no exterior abnormality was observed, and the interior material had a surface layer with a high transparency. The results of the deodorant capability test are summarized in Table 5; and the results of the antimicrobial capability test and the antifungal capability test are summarized in Table 6.

[Working example 3]

<Preparation of silver-zinc alloy particle mixed dispersion liquid>

An alloy particle dispersion liquid ($\gamma$) (Table 3) was obtained in a similar manner as the working example 1, except that there was used a raw material metal compound-containing solution (III) (Table 2) with ethylene glycol being a solvent, and with silver nitrate and a zinc nitrate hexahydrate being dissolved therein in a way such that a concentration as Ag was 3.75 mmol/L, and a concentration as Zn was 1.25 mmol/L.

The titanium oxide particle dispersion liquid (B) and the alloy particle dispersion liquid ($\gamma$) were then mixed together in a way such that a mass ratio of the particles in each dispersion liquid (titanium oxide particles/alloy particles) would become 1,000, thereby obtaining a titanium oxide-alloy particle mixed dispersion liquid (e-3).

A coating liquid for evaluation (E-3) was produced in a similar manner as the working example 1, except that there was used the titanium oxide-alloy particle mixed dispersion liquid (e-3) (Table 4).

<Application to floor tile>

A floor tile (vinyl chloride resin-based) used as an interior floor material was cut into pieces of sizes suitable for various tests, followed by using the air-spray gun to apply the coating liquid for evaluation (E-3) to the pieces in a similar manner as the working example 1. The pieces were then dried in an oven at 50°C for an hour to obtain the interior material of the present invention. The surface of the interior material was then visually observed at a distance of 20 cm under visible light. As a result, no exterior abnormality was observed, and the interior material had a surface layer with a high transparency. The results of the deodorant capability test are summarized in Table 5; and the results of the antimicrobial capability test and the antifungal capability test are summarized in Table 6.

[Working example 4]

<Preparation of copper-zinc alloy particle mixed dispersion liquid>

An alloy particle dispersion liquid ($\delta$) (Table 3) was obtained in a similar manner as the working example 1, except that there was used a raw material metal compound-containing solution (IV) (Table 2) with ethylene glycol being a solvent, and with a copper nitrate trihydrate and a zinc nitrate hexahydrate being dissolved therein in a way such that a concentration as Cu was 3.75 mmol/L, and a concentration as Zn was 1.25 mmol/L.

The titanium oxide particle dispersion liquid (B) and the alloy particle dispersion liquid ($\delta$) were then mixed together in a way such that a mass ratio of the particles in each dispersion liquid (titanium oxide particles/alloy particles) would become 300, thereby obtaining a titanium oxide-alloy particle mixed dispersion liquid (e-4).

A coating liquid for evaluation (E-4) was produced in a similar manner as the working example 1, except that there was used the titanium oxide-alloy particle mixed dispersion liquid (e-4) (Table 4).

<Application to interior film>

A PET film that had been subjected to corona surface treatment (product model number "LUMIRROR T60" by Toray Industries, Inc.) was cut into pieces of sizes suitable for various tests, followed by using a bar coater to apply the coating liquid for evaluation (E-4) to the film surfaces that had been subjected to corona surface treatment. The pieces were then dried in an oven at 80°C for 30 min to obtain the interior material of the present invention. The surface of the interior material was then visually observed at a distance of 20 cm under visible light. As a result, no exterior abnormality was observed, and the interior material had a surface layer with a high transparency. The results of the deodorant capability test are summarized in Table 5; and the results of the antimicrobial capability test and the antifungal capability test are summarized in Table 6.

[Working example 5]

<Preparation of silver-copper alloy particle mixed dispersion liquid>

An alloy particle dispersion liquid ($\varepsilon$) (Table 3) was obtained in a similar manner as the working example 1, except that when performing concentration and pure water washing with the aid of an ultrafiltration membrane having a molecular weight cut-off of 10,000 (microza by Asahi Kasei Chemicals Corporation), the amount of water used for washing with respect to the amount of the alloy particle dispersion liquid obtained eventually was reduced by 1/2 (from tenfold to five time volume).

The titanium oxide particle dispersion liquid (B) and the alloy particle dispersion liquid (ε) were then mixed together in a way such that a mass ratio of the particles in each dispersion liquid (titanium oxide particles/alloy particles) would become 100, thereby obtaining a titanium oxide-alloy particle mixed dispersion liquid (e-5).

A coating liquid for evaluation (E-5) was produced in a similar manner as the working example 1, except that there was used the titanium oxide-alloy particle mixed dispersion liquid (e-5) (Table 4).

<Application to melamine decorative board>

A melamine decorative board used as an interior partition board was cut into pieces of sizes suitable for various tests, followed by using the air-spray gun to apply the coating liquid for evaluation (E-5) to the pieces in a similar manner as the working example 1. The pieces were then dried in an oven at 50°C for three hours to obtain the interior material of the present invention. The surface of the interior material was then visually observed at a distance of 20 cm under visible light. As a result, no exterior abnormality was observed, and the interior material had a surface layer with a high transparency. The results of the deodorant capability test are summarized in Table 5; and the results of the antimicrobial capability test and the antifungal capability test are summarized in Table 6.

[Working example 6]

<Preparation of zinc-magnesium alloy particle mixed dispersion liquid>

An alloy particle dispersion liquid (ζ) (Table 3) was obtained in a similar manner as the working example 1, except that there was used a raw material metal compound-containing solution (V) (Table 2) with ethylene glycol being a solvent, and with a zinc nitrate hexahydrate and a magnesium nitrate hexahydrate being dissolved therein in a way such that a concentration as Zn was 3.75 mmol/L, and a concentration as Mg was 1.25 mmol/L.

The titanium oxide particle dispersion liquid (A) and the alloy particle dispersion liquid (ζ) were then mixed together in a way such that a mass ratio of the particles in each dispersion liquid (titanium oxide particles/alloy particles) would become 300, thereby obtaining a titanium oxide-alloy particle mixed dispersion liquid (e-6).

A coating liquid for evaluation (E-6) was produced in a similar manner as the working example 1, except that there was used the titanium oxide-alloy particle dispersion liquid (e-6) (Table 4).

<Application to wall tile>

A wall tile (ceramics-made) used as a wall material was cut into pieces of sizes suitable for various tests, followed by using the air-spray gun to apply the coating liquid for evaluation (E-6) to the pieces in a similar manner as the working example 1. The pieces were then dried in an oven at 90°C for two hours to obtain the interior material of the present invention. The surface of the interior material was then visually observed at a distance of 20 cm under visible light. As a result, no exterior abnormality was observed, and the interior material had a surface layer with a high transparency. The results of the deodorant capability test are summarized in Table 5; and the results of the antimicrobial capability test and the antifungal capability test are summarized in Table 6.

[Comparative example 1]

A titanium oxide particle dispersion liquid (c-1) was obtained only from the dispersion liquid of the titanium oxide particles (A).

A coating liquid for evaluation (C-1) was produced in a similar manner as the working example 1, except that the titanium oxide particle dispersion liquid (c-1) was used (Table 4).

<Application to decorative gypsum board>

A sample(s) for performance evaluation were prepared in a similar manner as the working example 1, except that the coating liquid for evaluation (C-1) was used. The surface of the sample was then visually observed at a distance of 20 cm under visible light. As a result, no exterior abnormality was observed, and the sample had a surface layer with a high transparency. The results of the deodorant capability test are summarized in Table 5; and the results of the antimicrobial capability test and the antifungal capability test are summarized in Table 6.

[Comparative example 2]

An alloy particle dispersion liquid (c-2) was obtained only from the alloy particle dispersion liquid ($\alpha$).
A coating liquid for evaluation (C-2) was produced in a similar manner as the working example 1, except that the alloy particle dispersion liquid (c-2) was used (Table 4).

<Application to melamine decorative board>

A sample(s) for performance evaluation were prepared in a similar manner as the working example 2, except that the coating liquid for evaluation (C-2) was used. The surface of the sample was then visually observed at a distance of 20 cm under visible light. As a result, no exterior abnormality was observed, and the sample had a surface layer with a high transparency. The results of the deodorant capability test are summarized in Table 5; and the results of the antimicrobial capability test and the antifungal capability test are summarized in Table 6.

[Comparative example 3]

<Preparation of silver particle dispersion liquid>

With ethylene glycol being used as a solvent, there was obtained a raw material metal compound-containing solution (VI) (Table 2) with silver nitrate being dissolved therein in a way such that a concentration as silver was 4.00 mmol/L.
A silver particle dispersion liquid ($\eta$) (Table 3) was obtained in a similar manner as the working example 1, except that the raw material metal compound-containing solution (VI) was used.
The titanium oxide particle dispersion liquid (A) and the silver particle dispersion liquid ($\eta$) were then mixed together in a way such that a mass ratio of the particles in each dispersion liquid (titanium oxide particles/silver particles) would become 1,000, thereby obtaining a titanium oxide-silver particle dispersion liquid (c-3).
A coating liquid for evaluation (C-3) was produced in a similar manner as the working example 1, except that the titanium oxide-silver particle dispersion liquid (c-3) was used (Table 4).

<Application to floor tile>

A sample(s) for evaluation were prepared in a similar manner as the working example 3, except that the coating liquid for evaluation (C-3) was used. The surface of the sample was then visually observed at a distance of 20 cm under visible light. As a result, no exterior abnormality was observed, and the sample had a surface layer with a high transparency. The results of the deodorant capability test are summarized in Table 5; and the results of the antimicrobial capability test and the antifungal capability test are summarized in Table 6.

[Comparative example 4]

<Preparation of raw material silver liquid>

With a pure water being used as a solvent, there was obtained a raw material silver compound-containing solution (VII) (Table 2) with silver nitrate being dissolved therein in a way such that a concentration as silver was 4.00 mmol/L.
The raw material silver compound-containing solution (VII) was then mixed into the titanium oxide particle dispersion liquid (A) in a way such that a mass ratio of the particles in each dispersion liquid (titanium oxide particles/silver component) would become 300, thereby obtaining a titanium oxide-silver particle dispersion liquid (c-4). The titanium oxide particles in the titanium oxide-silver particle dispersion liquid agglutinated.
A coating liquid for evaluation (C-4) was produced in a similar manner as the working example 1, except that the titanium oxide-silver particle dispersion liquid (c-4) was used (Table 4).

<Application to melamine decorative board>

A sample(s) for performance evaluation were prepared in a similar manner as the working example 2, except that the coating liquid for evaluation (C-4) was used. The surface of the sample was then visually observed at a distance of 20 cm under visible light. As a result, white turbidity was confirmed on its outer appearance; the sample did not have a surface layer with a high transparency. The results of the deodorant capability test are summarized in Table 5; and the results of the antimicrobial capability test and the antifungal capability test are summarized in Table 6.

[Table 1]

| Titanium oxide particle dispersion liquid | Non-volatile content concentration (% by mass) | Average particle size $D_{50}$ (nm) | Crystalline phase |
|---|---|---|---|
| (A) | 1.00 | 12 | Anatase |
| (B) | 1.00 | 9 | Rutile |

[Table 2]

| Raw material metal compound-containing solution | Solvent 1 | Alloy component 1 | Concentration (mmol/L) | Alloy component 2 | Concentration (mmol/L) | Ratio of antimicrobial metal (% by mass) |
|---|---|---|---|---|---|---|
| (I) | Ethylene glycol | $AgNO_3$ | 2.50 | $Cu(NO_3)_2 \cdot 3H_2O$ | 2.50 | 100 |
| (II) | Pure water | $AgNO_3$ | 4.50 | $Pd(NO_3)_2 \cdot 2H_2O$ | 0.50 | 100 |
| (III) | Ethylene glycol | $AgNO_3$ | 3.75 | $Zn(NO_3)_2 \cdot 6H_2O$ | 1.25 | 100 |
| (IV) | Ethylene glycol | $Cu(NO_3)_2 \cdot 3H_2O$ | 3.75 | $Zn(NO_3)_2 \cdot 6H_2O$ | 1.25 | 100 |
| (V) | Ethylene glycol | $Zn(NO_3)_2 \cdot 6H_2O$ | 3.75 | $Mg(NO_3)_2 \cdot 6H_2O$ | 1.25 | 75 |
| (VI) | Ethylene glycol | $AgNO_3$ | 4.00 | - | - | 100 |
| (VII) | Pure water | $AgNO_3$ | 4.00 | | | 100 |

[Table 3]

| Alloy particle dispersion liquid | Non-volatile content concentration (% by mass) | Alloy particle concentration (% by mass) | Alloy particle / Non-volatile impurity | Average particle size $D_{50}$ (nm) | Alloy determination by STEM |
|---|---|---|---|---|---|
| ($\alpha$) | 0.70 | 0.20 | 0.40 | 60 | o |
| ($\beta$) | 0.65 | 0.10 | 0.18 | 53 | o |
| ($\gamma$) | 0.60 | 0.08 | 0.15 | 45 | o |
| ($\delta$) | 0.60 | 0.08 | 0.15 | 49 | o |
| ($\epsilon$) | 0.70 | 0.05 | 0.08 | 30 | o |
| ($\zeta$) | 0.60 | 0.08 | 0.15 | 68 | o |
| ($\eta$) | 0.70 | 0.10 | 0.17 | 75 | × |

[Table 4]

| Coating liquid for evaluation | Titanium oxide particle dispersion liquid | Alloy particle dispersion liquid | Titanium oxide particle / Alloy particle | Non-volatile content concentration (% by mass) | Average particle size $D_{50}$ (nm) |
|---|---|---|---|---|---|
| (E-1) | (A) | (α) | 100 | 1.0 | 18 |
| (E-2) | (B) | (β) | 200 | 1.0 | 15 |
| (E-3) | (B) | (γ) | 1000 | 1.0 | 12 |
| (E-4) | (B) | (δ) | 300 | 1.0 | 13 |
| (E-5) | (B) | (ε) | 100 | 0.8 | 16 |
| (E-6) | (A) | (ζ) | 300 | 1.0 | 20 |
| (C-1) | (A) | - | - | 1.0 | 12 |
| (C-2) | - | (α) | - | 0.7 | 60 |
| (C-3) | (A) | (η) | 1000 | 1.0 | 22 |
| (C-4) | (A) | (VII) | 300 | 0.9 | 56 |

[Table 5]

| | Odorous component decline rate (%) | | | | | | | | | | Evaluation | |
|---|---|---|---|---|---|---|---|---|---|---|---|---|
| | Ammonia | Acetic acid | Hydrogen sulfide | Methyl mercaptan | Trimethylamine | Acetaldehyde | Pyridine | Isovaleric acid | Nonenal | Indole | Number of components showing decline rate of not lower than 30% | Grade |
| Working example 1 | 41 | 47 | 36 | 21 | 48 | 10 | 40 | 51 | 46 | 60 | 8 | A |
| Working example 2 | 38 | 44 | 35 | 14 | 43 | 9 | 41 | 44 | 46 | 53 | 8 | A |
| Working example 3 | 37 | 41 | 31 | 10 | 40 | 6 | 38 | 41 | 39 | 43 | 8 | A |
| Working example 4 | 35 | 42 | 29 | 9 | 42 | 7 | 39 | 42 | 40 | 45 | 7 | A |
| Working example 5 | 26 | 37 | 25 | 9 | 36 | 4 | 27 | 37 | 35 | 38 | 5 | B |
| Working example 6 | 35 | 37 | 29 | 11 | 35 | 8 | 28 | 39 | 36 | 42 | 6 | B |
| Comparative example 1 | 28 | 37 | 28 | 15 | 40 | 6 | 39 | 43 | 40 | 43 | 6 | B |
| Comparative example 2 | 10 | 18 | 8 | 5 | 10 | 3 | 18 | 20 | 18 | 16 | 0 | D |
| Comparative example 3 | 29 | 36 | 28 | 10 | 36 | 5 | 24 | 36 | 26 | 33 | 4 | C |
| Comparative example 4 | 22 | 35 | 26 | 8 | 28 | 6 | 27 | 34 | 27 | 32 | 3 | C |

[Table 6]

| | Antimicrobial capability test | | | Antifungal capability test | | | |
|---|---|---|---|---|---|---|---|
| | Antimicrobial activity value | | Grade | Fungus growth status (4 weeks) | Grade | Fungus growth status (8 weeks) | Grade |
| | E. coli | Staphylococcus aureus | | | | | |
| Working example 1 | 5.2 | 4.6 | A | 0 | A | 1 | A |
| Working example 2 | 5.0 | 4.5 | A | 0 | A | 1 | A |
| Working example 3 | 4.6 | 4.1 | A | 1 | A | 2 | B |
| Working example 4 | 4.5 | 4.0 | A | 1 | A | 2 | B |
| Working example 5 | 3.9 | 3.3 | B | 2 | B | 3 | B |
| Working example 6 | 4.1 | 3.6 | B | 2 | B | 2 | B |
| Comparative example 1 | 0.0 | 0.0 | c | 4 | C | 5 | C |
| Comparative example 2 | 4.8 | 4.6 | A | 3 | B | 4 | C |
| Comparative example 3 | 3.9 | 2.4 | B | 3 | B | 4 | C |
| Comparative example 4 | 4.1 | 2.6 | B | 4 | C | 4 | C |

As can be seen from the working examples 1 to 6, a deodorant property, an antimicrobial property and an antifungal property were exhibited by the particle mixture of the two kinds of particles which were the titanium oxide particles and the alloy particles containing the antimicrobial metals.

As can be seen from the comparative example 1, an antimicrobial property was not exhibited when using only the titanium oxide particle dispersion liquid.

As can be seen from the comparative example 2, a deodorant property was not exhibited when using only the alloy particle dispersion liquid.

As can be seen from the comparative example 3, a weak deodorant property and a weak antifungal property were exhibited when using the titanium oxide-silver particle dispersion liquid comprised of the mixture of the titanium oxide particles and the silver particles.

As can be seen from the comparative example 4, as a result of adding the silver solution to the titanium oxide particles, the transparency deteriorated as the particle size of the titanium oxide particles in the titanium oxide particle dispersion liquid grew larger, and a weak deodorant property and a weak antifungal property were exhibited as well.

Based on these results, it can be seen that the interior material of the present invention is capable of controlling unpleasant odors and preventing contamination by microorganisms such as bacteria and fungi (molds), thus making it possible to keep the living space clean.

**Claims**

1.  An interior material having a surface layer containing (i) titanium oxide particles and (ii) antimicrobial metal-containing alloy particles.

2.  The interior material according to claim 1, wherein an antimicrobial metal(s) contained in the (ii) antimicrobial metal-containing alloy particles is at least one kind of metal selected from the group consisting of silver, copper and zinc.

3. The interior material according to claim 2, wherein the (ii) antimicrobial metal-containing alloy particles at least contain silver.

4. The interior material according to any one of claims 1 to 3, wherein the antimicrobial metal(s) contained in the (ii) antimicrobial metal-containing alloy particles is in an amount of 1 to 100% by mass with respect to a total mass of the alloy particles.

5. The interior material according to any one of claims 1 to 4, wherein a dispersed particle size of a particle mixture of the (i) titanium oxide particles and the (ii) antimicrobial metal-containing alloy particles is 5 to 100 nm in terms of a 50% cumulative distribution diameter $D_{50}$ on volume basis that is measured by a dynamic light scattering method using a laser light.

6. The interior material according to any one of claims 1 to 5, wherein the surface layer further contains a binder.

7. The interior material according to claim 6, wherein the binder is a silicon compound-based binder.

8. The interior material according to any one of claims 1 to 7, wherein the interior material is a material selected from the group consisting of an interior architectural material, a vehicular interior material, a material for household furniture and a material for electric appliances.

9. A method for producing the interior material according to claim 1, comprising a step of applying a dispersion liquid containing the (i) titanium oxide particles and the (ii) antimicrobial metal-containing alloy particles to a surface of the interior material.

10. The method for producing the interior material according to claim 9, wherein the dispersion liquid containing the (i) titanium oxide particles and the (ii) antimicrobial metal-containing alloy particles is applied via spray coating, flow coating, dip coating, spin coating, Meyer bar coating, gravure coating, knife coating, kiss coating, die coating and/or film transfer.

## INTERNATIONAL SEARCH REPORT

| | International application No. |
|---|---|
| | PCT/JP2019/012716 |

**A. CLASSIFICATION OF SUBJECT MATTER**

Int. Cl. A61L9/01(2006.01)i, A01N59/16(2006.01)i, A01N59/20(2006.01)i, B60R13/02(2006.01)i, C08J7/04(2006.01)i, C09D5/14(2006.01)i, C09D7/61(2018.01)i, C09D183/00(2006.01)i, C09D201/00(2006.01)i, E04F13/08(2006.01)i

According to International Patent Classification (IPC) or to both national classification and IPC

**B. FIELDS SEARCHED**

Minimum documentation searched (classification system followed by classification symbols)

Int. Cl. A61L9/01, A01N59/16, A01N59/20, B60R13/02, C08J7/04, C09D5/14, C09D7/61, C09D183/00, C09D201/00, E04F13/08

Documentation searched other than minimum documentation to the extent that such documents are included in the fields searched

Published examined utility model applications of Japan       1922-1996
Published unexamined utility model applications of Japan     1971-2019
Registered utility model specifications of Japan             1996-2019
Published registered utility model applications of Japan     1994-2019

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2000-15110 A (NISSHIN STEEL CO., LTD.) 18 January 2000, claims, paragraphs [0010], [0019], examples 1-4 (Family: none) | 1-5, 8<br>5-10 |
| X<br>Y | JP 11-228306 A (NISSHIN STEEL CO., LTD.) 24 August 1999, claims, paragraphs [0013], [0014], [0017], [0027], fig. 3, 4, example 1 (Family: none) | 1-5, 8<br>5-10 |
| X<br>Y | JP 2002-345933 A (NIHON TECHNICAL DEVELOPMENT CENTER CO., LTD.) 03 December 2002, claims, paragraphs [0001], [0025], [0032]-[0036], [0043] (Family: none) | 1-2, 4, 6, 8<br>5-10 |

☒ Further documents are listed in the continuation of Box C.      ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07.06.2019 | 18.06.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## INTERNATIONAL SEARCH REPORT

| International application No. |
| --- |
| PCT/JP2019/012716 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2014-80851 A (TOSHIBA CORP.) 08 May 2014, claims, paragraphs [0002], [0058], [0059] & US 2010/0204041 A1, claims, paragraphs [0002], [0062], [0063] & WO 2009/031316 A1 & EP 2186561 A1 & CN 101795767 A | 6-10 |
| Y | JP 2000-317313 A (SHARP CORP.) 21 November 2000, claims, paragraphs [0021], [0029], [0043] (Family: none) | 6-10 |
| Y | JP 2008-179660 A (MITSUBISHI ELECTRIC CORP.) 07 August 2008, claims, paragraphs [0001], [0010], [0023], [0027] (Family: none) | 6-10 |
| A | JP 2007-212125 A (MATSUSHITA ELECTRIC INDUSTRIAL CO., LTD.) 23 August 2007, claims, paragraph [0042] & JP 2012-247185 A | 1-10 |
| E, X | JP 2019-63143 A (SHIN-ETSU CHEMICAL CO., LTD.) 25 April 2019, claims, paragraphs [0007], [0074]-[0079], examples (in particular, paragraphs [0140]-[0144]) & EP 3461333 A1, claims, paragraphs [0007], [0091]-[0097], examples (in particular, paragraphs [0160]-[0164]) & US 2019/0099510 A1 & CN 1099566652 A & KR 10-2019-0038380 A | 1-10 |
| E, X | JP 2019-63712 A (SHIN-ETSU CHEMICAL CO., LTD.) 25 April 2019, claims, paragraphs [0077]-[0082], examples & EP 3461336 A1, claims, paragraphs [0094]-[0101], examples & US 2019/0099741 A1 & CN 109566648 A & KR 10-2019-0038379 A | 1-10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003533588 PCT
- JP 2003113392 A
- JP 2001070423 A
- JP 2001037861 A
- JP 2001178806 A
- JP 2005318999 A

**Non-patent literature cited in the description**

- **MIYANO.** *Iron and steel,* 2007, vol. 93 (1), 57-65
- **H. KAWAKAMI.** *ISIJ Intern.,* 2008, vol. 48 (9), 1299-1304